# EUROPEAN PATENT SPECIFICATION

(11) **EP 0 508 918 B1**
(45) Date of publication and mention of the grant of the patent: **31.01.1996**
(21) Application number: 92420163.5
(22) Date of filing: 29.07.1988
(51) Int. Cl.: B01J 23/60, B01J 37/18, C07C 29/149, C07C 29/145, C07C 29/141

(54) **Method for production of alcohols by low pressure hydrogen- ation of carbonyl-containing compounds using palladium on zinc oxide catalysts.**
Verfahren zur Herstellung von Alkoholen durch Niederdruck- hydrierung von Carbonylhaltigen Zusammensetzungen mittels Palladium auf Zinkoxid enthaltenden Katalysatoren.
Procédé de production d'alcools par hydrogénation à basse pression de composés contenant du carbonyle en utilisant des catalyseurs à base de palladium sur l'oxyde de zinc.

(30) Priority: 03.08.1987 US 81252; 01.02.1988 US 150791; 15.07.1988 US 219630
(43) Date of publication of application: 14.10.1992
(62) Divisional of application: 88908047.9
(73) Proprietor: EASTMAN CHEMICAL COMPANY, Kingsport, TN 37660 (US)
(72) Inventor: Gustafson, Bruce Leroy, c/o EASTMAN KODAK COMPANY, Rochester, New York 14650-2201 (US); Wehner, Paul Sherman, c/o EASTMAN KODAK COMPANY, Rochester, New York 14650-2201 (US); Mercer, Patricia Lee Nixon, c/o EASTMAN KODAK CO., Rochester, New York 14650-2201 (US); Nelson, Gregory Otis, c/o EASTMAN KODAK COMPANY, Rochester, New York 14650-2201 (US)
(74) Representative: Behrens, Dieter, Dr.-Ing.

(56) References cited:
- EP-A- 0 126 552
- EP-A- 0 241 760
- FR-A- 1 392 376
- US-A- 4 052 467
- US-A- 4 518 714

## Description

This invention relates to a process for the selective reduction of carbonyl-containing compounds to alcohols.

The catalytic hydrogenation of carbonyl-containing compounds, e.g., esters, to produce their corresponding alcohols, is potentially of great commercial value. Catalysts traditionally employed for such conversions include copper chromite based materials, frequently containing a promoter such as barium. Unfortunately, these catalysts typically require high pressure to achieve commercially attractive reaction rates for the hydrogenation of esters, i.e., pressures in excess of 3000 psig (21000 kPa). In addition, chromium and barium present toxicity and environmental concerns which must be dealt with if one is to economically and safely use these materials on a commercial scale.

More recently, substantial amounts of research have been carried out in efforts to develop hydrogenation catalysts capable of reducing carbonyl-containing compounds, e.g., organic acids and esters, to alcohols at reduced pressures. While such catalysts are capable of promoting the hydrogenation of carbonyl-containing compounds to produce alcohols, one problem with such materials is the need to run at very low liquid hourly space velocities in order to achieve suitably high conversion levels.

Another problem frequently encountered with such prior art low pressure catalyst systems when employed for the reduction of carbonyl-containing compounds such as aldehydes and ketones, is their lack of selectivity to the desired alcohol product, such catalysts frequently being too active and thus producing product which results from reaction of substrate with additional hydrogen.

Yet another problem encountered with such prior art low pressure catalyst systems, such as Raney nickel, is the ease of handling of such catalysts, which are frequently pyrophoric, and thus require special handling to avoid fire hazard.

### Objects of the Invention

An object of the present invention, therefore, is a process for the low pressure, high selectivity, high activity hydrogenation of carbonyl-containing compounds to produce alcohols.

Another object of the present invention is a catalyst system which is capable of promoting the hydrogenation of carbonyl-containing compounds at low reaction pressures.

Still another object of the present invention is a catalyst system which is capable of promoting the hydrogenation of carbonyl-containing compounds at low reaction pressure, which catalyst system is readily prepared and requires no special handling precautions.

These and other objects of the present invention will become apparent from inspection of the detailed description and the appended claims which follow.

### Statement of the Invention

In accordance with the present invention, it has been discovered that palladium supported on zinc oxide is an effective catalyst for the low pressure hydrogenation of carbonyl-containing compounds to selectively produce alcohols in high yield.

The invention hydrogenation process employs readily prepared, easily handled catalysts and enables a commercially important reaction, i.e., the conversion of carbonyl-containing compounds to alcohols, to be carried out at low reaction pressures, thereby reducing the cost of equipment required for the desired hydrogenation reaction and reducing the safety risks involved in such conversions.

### Detailed Description of the Invention

In accordance with the present invention, there is provided a process for the low pressure hydrogenation of carbonyl-containing compounds of specified structure to produce the corresponding alcohols, which process comprises contacting the carbonyl-containing compounds with a catalyst comprising 0.01 up to 20 weight percent palladium on a zinc oxide-containing support in the presence of hydrogen under hydrogenation conditions, and in the further presence of in the range of 0,01 up to 2,0 wt % water, based on the total weight of reactants and solvent charged to the reactor.

Catalysts employed in one embodiment of the present invention comprise palladium on zinc oxide support. A wide variety of techniques for contacting palladium and zinc oxide are suitable. For example, palladium can be applied directly to preformed zinc oxide employing such techniques as incipient wetness, wet impregnation, metal atom evaporation, precipitation, or appropriate precursors of palladium and zinc can be coprecipitated, then calcined to remove the counter ions introduced by the precursor compounds, and, finally, reduced to convert the palladium to an active form.

A wide range of zinc compounds are suitable zinc oxide precursor for use in the practice of the present invention, e.g., zinc nitrate, zinc halides, zinc acetate and zinc carbonate.

When zinc oxide is employed as catalyst support, a variety of zinc oxide compounds can be directly employed as catalyst support. Typically, such preformed zinc oxide materials will contain an impurity content no greater than about 5 %. Preferred zinc oxide compounds employed as catalyst support will contain at least 60 % zinc oxide by weight, with up to 40 % by weight of inert materials such as :
SiO₂,
Al₂O₃, and
TiO₂
being employed as inert diluents and as catalyst binders.

The surface area of the catalyst supports employed in the practice of the present invention can vary widely. Preferably, support materials employed in the practice of the invention will have surface areas of at least about 1 m²/g. Of course, those of skill in the art also recognize that higher surface area materials will generally produce higher activity catalysts than lower surface area catalysts having comparable composition.

When zinc oxide support is prepared by calcination of a precipitated zinc oxide precursor, temperatures in the range of 200 up to 400°C are generally employed. Such temperature is maintained for a time sufficient to remove substantially all the counter ions introduced by the zinc oxide precursor (and the palladium compound employed) to form the catalyst. Times in the range of 2 up to 8 hours or longer are generally effective for this purpose.

Suitable sources of palladium are any compounds which are reducible when subjected to reducing conditions. Since many palladium compounds are convertible to the oxide form upon calcination under the above-described conditions, and the oxides of palladium are readily reduced, many palladium compounds are useful for catalyst preparation. Exemplary palladium compounds include the palladium halides, palladium acetate, palladium nitrate, palladium ammine complexes, organometallic complexes of palladium.

The term "carbonyl-containing compounds" as employed in this specification is intended to include compounds of the structure 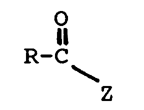
wherein R is a C₁-C₂₀ alkyl or substituted alkyl radical ; or
a C₂-C₂₀ alkenyl radical or substituted derivative thereof ;
wherein said substituted groups include ethers, amines, additional carbonyl groups, aryl groups, hydroxyl groups and alkoxy groups ; and
Z=H,
- R',: wherein R' is defined the same as R, and is selected independently of R,
- OR',: wherein R' is as defined above,
- X,: wherein X is any one of the halogens, or
- NR''₂,: wherein each R'' is independently selected from H or R';
with the proviso that R and Z can be joined as part of a polymethylene or hydrocarbyl- or heteroatom-substituted polymethylene radical,
poly-carbonyl analogs of such carbonyl-containing compounds; and
mixtures of any two or more thereof.

Preferred carbonyl-containing compounds are compounds selected from the group consisting of:

YO₂C―A―CO₂Y , (a)

wherein A is an alkylene moiety, an alkenylene moiety, or an alkynylene moiety having 1 up to 20 carbon atoms, or substituted derivative thereof, or a cycloalkyl or cycloalkenyl moiety having 4-12 carbon atoms or substituted derivative thereof; and wherein each Y is independently a C₁ up to C₁₂ alkyl, alkenyl or alkynyl radical or substituted derivative thereof;

B-CO₂Y (b)

wherein B is an alkyl, alkenyl or alkynyl radical, or substituted derivative thereof, having 1 up to 20 carbon atoms; and
wherein Y is defined as above; 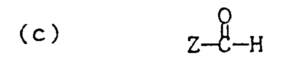
wherein Z is an alkyl, alkenyl or alkynyl radical having 1 up to 20 carbon atoms or substituted derivatives thereof; and
mixtures of any two or more thereof.

Exemplary carbonyl-containing compounds which satisfy the above formulae include alkyl oleates, dialkyl adipates, propionaldehyde, dialkyl cyclohexane dicarboxylates, alkyl acrylates, alkyl propionates, alkyl isobutyrates, alkyl normal butyrates, alkyl acetates, nonanal, dialkyl butane dicarboxylates, alkyl methacrylates, alkyl crotonates, alkyl isocrotonates, alkyl sorbates, alkyl cinnamates, maleic anhydride, alkyl fumarates, dialkyl succinates, succinic anhydride, alkyl glutarates, dialkyl malonates, dialkyl octanedioates, dialkyl decanedioates, dialkyl dodecanedioates, alkyl laurates, alkyl myristates, alkyl palmitates, alkyl stearates, alkyl linoleates, alkyl linolenates, alkyl isovalerates, alkyl normal valerates, alkyl caproates, alkyl caprylates, alkyl 2-ethylhexanoates, dialkyl cyclohexanedioates, γ-butyrolactone, alkyl phenylacetates, alkyl cyclohexane carboxylates, alkyl pyruvates, alkyl glycolates, alkyl oxalates, alkyl formates, alkyl lactates, alkyl citrates and glyceride esters.

Typical alkyl groups employed have from 1 up to 20 carbon atoms, with alkyl groups having 1 up to 6 carbon atoms being preferred.

The hydrogenation process of the present invention involves contacting at least one of the above-described carbonyl-containing compounds with at least one of the above-described palladium/zinc oxide catalysts in the presence of hydrogen under hydrogenation condtions. Hydrogenation conditions typically employed in the practice of the present invention are set forth below.

The process of the present invention can be operated in a variety of configurations. Depending on the substrate to be hydrogenated, the preferred method of opeation is frequently in a fixed bed flow reaction system. If the vapor pressure of the substrate to be hydrogenated is sufficiently high at reaction temperature, the desired method of operation may be vapor phase, i.e., all reactants and products exist in the gaseous phase. For other substrates, the desired method of operation may be a trickle bed configuration. Regardless of the method of operation, the desired time of contact between the reactants and catalyst components can be varied as desired to achieve the desired level of reaction.

In typical fixed bed operation, pressures in the range of 100-10,000 psig will be employed (700 - 70 000 kPa). Preferably the pressure will be in the range of 100-2500 psig (700 - 17 500 kPa). Similarly, temperatures in the range of 25-400°C can be used, with a more perferred range of 100-290°C. While the feed rate of the substrate will be varied to control the level of conversion, normal liquid hourly space velocities (LHSV) will be in the range of 0.01-100 h⁻¹, with a preferred range of 0.1-20 h⁻¹. The molar ratio of hydrogen to substrate will typically be in the range of 1:1 to 1000:1 with a preferred range of 2:1 to 100:1.

Alternatively the invention may be conducted in a slurry phase reactor. In slurry phase operation, the ratio of carbonyl-containing compound to catalysts employed can vary widely, with ratios as low as 1:1 or lower being operable, but not economically attractive; and ratios as high as 10,000:1 and higher also being operable, but generally providing relatively low conversions unless very long contact times are employed. Preferred carbonyl-containing compound:catalysts ratios fall within the range of 1:1 up to 1,000:1, with ratios in the range of 2:1 up to 100:1 being most preferred because good levels of conversion of the carbonyl-containing compounds are obtained without requiring excessive amounts of catalysts, or extremely long contact times.

While the invention hydrogenation process can be carried out in the absence of solvent, it is presently preferred to perform the process in the presence of a suitable solvent. Suitable solvents are compounds which are fluid and in which the carbonyl-containing starting material is soluble at reaction temperature, and which are non-reactive under hydrogenation conditions. Preferred solvents are those which are fluid and in which the carbonyl-containing starting material is soluble at room temperature. Exemplary solvents include aromatic solvents such as toluene; alcohols such as methanol; ethers such as diphenyl ether and tetrahydrofuran.

When employed, the volume/volume ratio of solvent to substrate can vary widely, typically falling in the range of 5:95 to 95:5.

In a preferred embodiment of the present invention, hydrogenation of carbonyl-containing compounds is carried out with small amounts of water (i.e., 0.01 up to 2 wt. % water based on the total weight of reactants and solvent) present in the reaction mixture. It has been found that selectivity to hydrogenation (as opposed to transesterification between reactant and product) products is greatly improved by the presence of such small quantities of water in the reaction mixture.

Following hydrogenation, the desired product can be recovered and purified using conventional techniques well known to those of skill in the art. For example, catalysts can be removed from the reaction mixture by filtration, decantation and the like. By-products and unreacted starting material as well as solvent, if employed, can be separated from the product by distillation, recrystallization, or solvent/solvent extraction, for example.

The invention will now be described in greater detail by reference to the following non-limiting examples.

### EXAMPLE 1 - Catalyst Preparation

### Pd on ZnO Support

A sample of 1 wt % Pd supported on ZnO was prepared as follows: A solution of Pd was prepared by dissolving 20 g of Pd(NO₃)₂ in 50 ml of H₂O and 16 g of HNO₃. This solution was diluted to 750 ml and added to 925 g of powdered ZnO. The resulting mixture was heated at 90°C in air until dry. The sample was then calcined at 200°C for two hours prior to catalytic evaluation. Approximately 1 cc of powdered sample was loaded into a plug flow micro-reactor and heated to 300°C in flowing hydrogen. The catalyst was held at 300°C for two hours prior to evaluation for hydrogenation of carbonyl-containing compounds.

### EXAMPLE 2 - Methyl Acetate Hydrogenation ; Effect of Water in the Feed

Catalyst prepared as described in Example 1 was employed for the vapor phase hydrogenation of methyl acetate. All evaluations were conducted using a hydrogen/ester ratio of 4.0 and a gas hourly space velocity (GHSV; volume of gas/volume of catalyst/hr) of 30,180 hr⁻¹. The effect of water content in the feed is demonstrated by the results presented in Table I.

**Table I**

| Catalytic Hydrogenation over Pd/ZnO | | | | | | |
|---|---|---|---|---|---|---|
| Run # | Temp., °C | Pressure, psig (kPa) | wt % H₂O | Rate (µmoles/ g-cat sec)* | | |
| | | | | MeOH | EtOH | EtOAc |
| 1 | 298 | 730 (5110) | 0.0 | 7.3 | 2.0 | 3.6 |
| 2 | 298 | 720 (5040) | 0.1 | 4.3 | 2.4 | 1.8 |
| 3 | 298 | 720 (5040) | 0.5 | 2.8 | 1.5 | 0.3 |
| 4 | 296 | 725 (5075) | 1.0 | 1.5 | 1.1 | 0.1 |

| | | | | | | |
|---|---|---|---|---|---|---|
| *MeOH = methanol EtOH = ethanol EtOAc = ethyl acetate | | | | | | |

The results set forth in Table I demonstrate that the addition of small amounts of water to the reaction mixture promotes improved catalyst performance by reducing the occurrence of undesired transesterification by-product formation.

### EXAMPLE 3 - Hydrogenation of Methyl Acetate

### a. Pd on ZnO Catalyst

Catalyst prepared as described in Example 1 was evaluated in the manner described in Example 2. The water content of the feed was approximatively 1 % by weight for all of the runs carried out. Results from these evaluations are presented in Table II.

**TABLE II**

| Catalyst Evaluation of Pb/ZnO Methyl Acetate Hydrogenation | | | | | | |
|---|---|---|---|---|---|---|
| Run # | Temp. °C, | Press psig(kPa) | H2/Ester moles | Rate (µmoles/ g-cat sec)* | | |
| | | | | MeOH | EtOH | EtOAc |
| 5 | 296 | 570 (3990) | 0.34 | 2.1 | 1.4 | <0.01 |
| 6 | 295 | 735 (5145) | 0.34 | 2,5 | 1,8 | 0,05 |
| 7 | 307 | 980 (6860) | 0.15 | 7,1 | 5,9 | 0,7 |
| 8 | 333 | 735 (5145) | 3.97 | 11.0 | 9.0 | 0.8. |

| | | | | | | |
|---|---|---|---|---|---|---|
| * MeOH = methanol EtOH = ethanol EtOAc = ethyl acetate | | | | | | |

The results set forth in Table II demonstrate that Pd/Zno catalyst is effective for the conversion of esters with high selectivity to the corresponding alcohols at pressures below 1000 psig (7000 kpa). In addition, excellent catalyst performance is obtained under a variety of the reaction parameters, i.e., temperature, pressure and feed composition.

### EXAMPLE 4 - Hydrogenation of Methyl Propionate Pd/ZnO Catalyst

The catalyst prepared as described in Example 1 was evaluated in the same manner as described in Example 2 for the hydrogenation of methyl propionate. Reaction parameters and results from this evaluation are given below.

| | |
|---|---|
| Temperature | 333°C |
| Pressure | 710 psig (4970 kpa) |
| H₂/ester | 4.8 |
| GHSV | 29,178 h⁻¹ |
| Conversion | 16. % |

| | methanol | propanol |
|---|---|---|
| Rate (µmoles/g-cat sec) : | 12. | 9.0 |

These results demonstrate that Pd/ZnO is an effective catalyst for the hydrogenation of methyl propionate to propanol and methanol. Note the very high reaction rates obtained even under non-optimized reaction conditions.

### EXAMPLE 5 - Hydrogenation of Methyl n-Butyrate

The catalyst prepared as described in Example 1 was evaluated in the same manner as described in Example 2 for the hydrogenation of methyl n-butyrate. Reaction parameters and results from this evaluation are given below.

| | |
|---|---|
| Temperature | 333°C |
| Pressure | 725 psig (5075 kPa) |
| H₂/ester | 5.6 |
| GHSV | 28,410 h⁻¹ |
| Conversion | 17. % |

| | methanol | n-butanol |
|---|---|---|
| Rate (µmoles/g-cat sec): | 5.9 | 6.1 |

The results demonstrate that Pd/ZnO is an effective catalyst for the hydrogenation of methyl n-butyrate to n-butanol and methanol.

### EXAMPLE 6 - Hydrogenation of Methyl i-Butyrate

The catalyst prepared as described in Example 1 was evaluated in the same manner as described in Example 2 for the hydrogenation of methyl i-butyrate. Reaction parameters and results from this evaluation are given below.

| | |
|---|---|
| Temperature | 334°C |
| Pressure | 725 psig (5075 kPa) |
| H₂/ester | 5.7 |
| GHSV | 28,372 h⁻¹ |
| Conversion | 25. % |

| | methanol | i-butanol |
|---|---|---|
| Rate (µmoles/g-cat sec): | 2.9 | 6.0 |

These results demonstrate that Pd/ZnO is an effective catalyst for the hydrogenation of methyl i-butyrate to i-butanol and methanol. Even under these non-optimized reaction conditions, the per pass conversion was 25%.

### EXAMPLE 7 - Hydrogenation of Ethyl Acetate

### Pd/ZnO Catalyst

The catalyst prepared as described in Example 1 was evaluated in the same manner as described in Example 2 for the hydrogenation of ethyl acetate. Reaction parameters and results from this evaluation are given below.

| | |
|---|---|
| Temperature | 332°C |
| Pressure | 725 psig (5075 kPa) |
| H₂/ester | 4.9 |
| GHSV | 29,100 h⁻¹ |
| Conversion | 16. % |

| | Ethanol |
|---|---|
| Rate (µmoles/g-cat sec) : | 16.7 |

These results demonstrate that Pd/ZnO is an effective catalyst for the hydrogenation of ethyl acetate to ethanol. Note the high selectivity and very high rate of reaction even under these non-optimized conditions.

### EXAMPLE 8 - Hydrogenation of 1,4-Dumethylcyclohexane Dicarboxylate

A catalyst was prepared in the same manner as described in Example 1. For catalytic evaluation, 260 cc of catalyst (14X40 standard mesh particles) was loaded into a 1 inch (2.54 cm) fixed bed reactor. The sample was treated in a hydrogen flow while heating the catalyst to reaction temperature. The system pressure was then increased to the desired reaction pressure. The ester feed consisted of 10 wt% 1,4-dimethylcyclohexane dicarboxylate (DMCD) in 1-dodecanol. Catalytic evaluation was conducted using a liquid feed rate of 80 g/h, 290°C, 1250 psig, and a 1000 standard cubic centimeters (sccm) hydrogen flow. Under these conditions, the DMCD conversion was 93.3%. The observed products were 60 mol % cyclohexanedimethanol (CHDM) and 40 mole % 1-methyl, 4-dodecyl, cyclohexanedicarboxylate.

The above-described catalyst and reaction set-up were employed in the same manner as described except that the reaction conditions were changed to a liquid feed rate of 13 g/h, 300°C, 1250 psig (8750 kPa), and 130 sccm hydrogen. Under these conditions, the observed conversion of DMCD was 99.5% with 93.3% molar selectivity to CHDM.

These results demonstrate that Pd/ZnO is a very effective catalyst for the selective hydrogenation of DMCD to CHDM.

### EXAMPLE 9 - Effect of Support Surface Area on Catalyst Performance

Catalyst samples were prepared as described in Example 1 except that various sources of ZnO were used for the catalyst support.

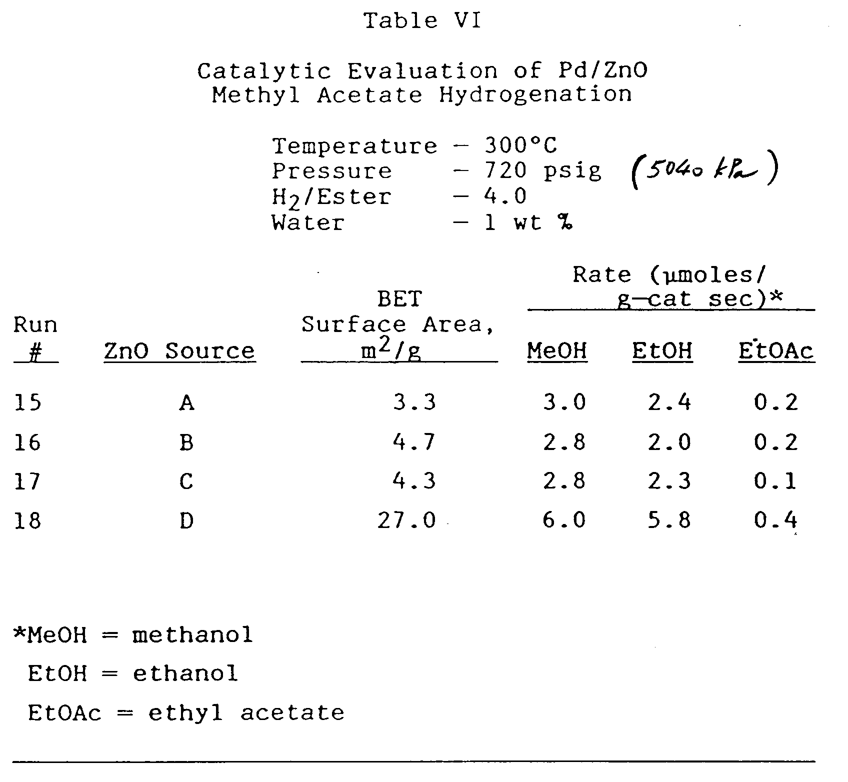

These results demonstrate that a variety of zinc oxide-containing support materials are useful supports for the Pd/ZnO catalyst of the present invention. In addition, the results show that the rate of reaction with Pd/ZnO catalyst is greatly improved with higher surface area support.

### EXAMPLE 10 - Catalyst with Modified Catalyst Support: ZnO/Al₂O₃

ZnO/Al₂O₃ was prepared by dissolving 365 g of zinc nitrate in 800 ml of water at 60°C. A solution of Na₂CO₃ (160 g/700 ml) was slowly added to the Zn solution while stirring to precipitate the Zn. The resulting precipitate was washed in 1000 ml of water, filtered and dried at 90°C. The resulting solid was heated in a flow of air at 350°C for four hours. A portion of this solid (50 g) was added to 32 g of aluminum hydrate and 200 ml of water. This mix was blended for 20 minutes, filtered, and finally heated in a flow of air at 350°C for four hours. The resulting powder had a BET surface area of 79 m²/g. The final Pd/ZnO, Al₂O₃ catalyst was prepared by adding an aqueous solution containing 0.24 g of Pd nitrate to 10 g of the ZnO/Al₂O₃ support material, drying the resulting mixture at 90°C, and then heating in a flow of air at 250°C for four hours.

The catalyst was evaluated for methyl acetate hydrogenation activity as described in Example 2. The reaction parameters employed and results obtained were as follows:

| | |
|---|---|
| Temperature | 332°C |
| Pressure | 720 psig (5040 kPa) |
| H₂/ester | 4.1 |
| Wt% H₂O | 0.0 |
| GHSV | 30,180 h⁻¹ |
| Methyl Acetate conversion | 12.0% |

| Product | Rate (µmoles/g-cat sec) |
|---|---|
| CH₃OH | 21.4 |
| CH₃CH₂OH | 5.6 |
| CH₃CO₂CH₂CH₃ | 8.8 |

These results indicate that alumina can be added to the preparation of invention catalyst, if desired. It is of note that high selectivity to the desired hydrogenation products, methanol and ethanol, are obtained even at the high rates of reaction achieved with the Pd/ZnO-Al₂O₃ catalyst.

### EXAMPLE 11 - Diethyl Adipate Hydrogenation

A hydrogenation reaction was conducted as described in Example 8 except that diethyl adipate was used in place of DMCD and the catalyst volume was 100 cc. Operation conditions are given below:

| | |
|---|---|
| Temperature | 300°C |
| Pressure | 1233 psig (8631 kPa) |
| H₂ Feed Rate | 760 sccm |
| Liquid Feed Rate | 189 ml/hr |

Analyses of the liquid product stream gave the following results:

| | |
|---|---|
| Dodecyl alcohol | 92% |
| Diethyl adipate | 2.2% |
| 1,6-hexanediol | 2.8% |
| C₆⁺ products | 3.% |

The results show that Pd/Zno is effective for the conversion of dibasic esters such as diethyl adipate to the corresponding diol.

### EXAMPLE 12 - Methyl Oleate Hydrogenation

### Pd/ZnO Catalyst

Hydrogenation of methyl oleate was carried out as described in Example 11 except that the liquid feed contained pure methyl oleate. The liquid feed rate was 68 ml/hr and the reaction temperature was 290°C. Analysis of the product mixture gave the following results.

| | |
|---|---|
| Methyl oleate | 4.1 wt % |
| Stearyl Alcohol | 42 wt % |
| Oleyl Alcohol | 50 wt % |

These results demonstrate the utility of Pd/ZnO for the conversion of methyl oleate to a mixture of C-18 alcohols at low pressure. Of particular note is the fact that a significant portion of the hydrogenation product is unsaturated product, oleyl alcohol. Isolation of this product is particularly noteworthy in view of the fact that the material has been subjected to reducing conditions in the presence of a catalytic material (i.e., Pd) which is generally quite effective for double bond hydrogenation. Thus, hydrogenation in accordance with the present invention is seen to be selective for reduction of carbon-oxygen double bonds (i.e., carbonyl bonds) relative to carbon-carbon double bonds.

### EXAMPLE 13 - Hydrogenation of Propionaldehyde

A Pd/ZnO catalyst prepared as described in Example 1 was evaluated for the vapor phase hydrogenation of propionaldehyde in a micro reactor system. Approximatively 1 cc of catalyst was charged to the reactor and pretreated as described on previous examples. The reaction parameters employed and results obtained were as follows :

| | |
|---|---|
| Temperature | 130°C |
| Pressure | 800 psig (5600 kPa) |
| H₂/Aldehyde | 4.0 |
| GHSV | 30,000 h-1 |
| Propionaldehyde conversion | 49.0 % |

| Product | Selectivity, wt % |
|---|---|
| CH3CH2CH2OH | 45 |
| 2-methyl-2-pentenal | 55 |

The results demonstrate the utility of Pd/ZnO catalyst for the conversion of propionaldehyde to propanol.

### EXAMPLE 14 - Hydrogenation of Nonanal

A 1 % Pd on ZnO catalyst prepared as described in Example 1 was evaluated for the slurry phase hydrogenation of nonanal. Nonanal (4g) was mixed with toluene (75 ml) and 1 % Pd/ZnO (1 g). This mixture was placed in a stirred autoclave and heated to 100°C under 100 psig (700 kPa) hydrogen pressure. The system was pressurized to 1500 psig (10500 kPa) and the mixture stirred for two hours. The autoclave was then cooled, vented and the resulting product mixture analyzed by gas chromatography (GC). For the comparative example, 1 g of a commercially available 1 % Pd/Al₂O₃ was substituted for the Pd/ZnO catalyst. The results were as follows :

| Catalyt | GC Analysis, wt % | | |
|---|---|---|---|
| | Nonanal | Nonanol | Unknown |
| 1 % Pd/ZnO | 0.9 | 99. | 0.0 |
| 1 % Pd/Al2O3 | 28.7 | 60.2 | 11.1 |

Pd/ZnO exhibits substantially higher activity and selectivity than Pd/Al₂O₃ under identical conditions for the hydrogenation of nonanal.

## Claims

1. A process for the low pressure hydrogenation of carbonyl-containing compounds to produce the corresponding alcohol, wherein said carbonyl-containing compounds have the structure : 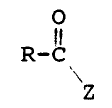 wherein R is a C₁-C₂₀ alkyl or substituted alkyl radical ; or
a C₂-c₂₀ alkenyl or alkynyl radical or a substituted derivative thereof ;
wherein said substituted groups include ethers, amines, additional carbonyl groups, aryl groups,
hydroxyl groups and alkoxy groups; and
Z = OR', wherein R' is defined the same as R, and is selected independently of R;
with the proviso that R and R' are different;
said process comprising contacting said carbonyl-containing compounds or mixtures of any two or more thereof with a zinc oxide supported palladium containing catalyst comprising 0,01 up to 20 wt % palladium calculated as the metal and based on the total weight of catalyst;
wherein said contacting is carried out in the presence of hydrogen under hydrogenation conditions and in the further presence of in the range of 0,01 up to 2,0 wt % water, based on the total weight of reactants and solvent charged to the reactor.

2. A process in accordance with Claim 1 wherein said hydrogenation conditions comprise a temperature in the range of 25 up to 400°C, and a pressure in the range of 100 up to 10,000 psig (700 - 70 000 kPa).

3. A process in accordance with Claim 1 wherein the hydrogen partial pressure falls within the range of 100 up to 10,000 psig (700 - 70 000 kPa).

4. A process in accordance with Claim 1 wherein said hydrogenation conditions comprise a temperature in the range of 100 up to 290°C and a pressure in the range of 100 up to 2500 psig (700 - 17 500 kPa)

5. A process in accordance with Claim 1 wherein the liquid hourly space velocity falls within the range of about 0.01 up to 100 h⁻¹.

6. A process in accordance with Claim 1 wherein the weight ratio of carbonyl-containing compound to catalyst falls within the range of 1:1 up to 10,000:1.

7. A process in accordance with Claim 1 wherein the carbonyl-containing compound is selected from the group consisting of :
YO₂C―A―CO₂Y, (a)
wherein A is an alkylene moiety, an alkenylene moiety, or an alkynylene moiety having 1 up to 20 carbon atoms, or substituted derivative thereof, or a cycloalkyl or cycloalkenyl moiety having 4-12 carbon atoms or substituted derivative thereof ; and wherein
each Y is independently a C₁ up to C₁₂ alkyl, alkenyl or alkynyl radical or substituted derivative thereof ;
B-CO₂Y (b)
wherein B is an alkyl, alkenyl or alkynyl radical, or substituted derivative thereof, having 1 up to 20 carbon atoms ; and
wherein Y is defined as above ; 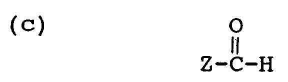 wherein Z is an alkyl, alkenyl or alkynyl radical having 1 up to 20 carbon atoms or substituted derivatives thereof ; and
mixtures of any two or more thereof.

8. A process in accordance with Claim 7 wherein the carbonyl-containing compound comprises a dialkyl adipate.

9. A process in accordance with Claim 7 wherein the carbonyl-containing compound comprises a dialkyl cyclohexanedicarboxylate.

10. A process in accordance with Claim 7 wherein the carbonyl-containing compound is selected from the group consisting of an alkyl oleate, an alkyl stearate, an alkyl linoleate, and alkyl linoleate, an alkyl α-eleostearate, an alkyl β-eleostearate, and mixtures of any two or more thereof.

11. A process in accordance with Claim 7 wherein the carbonyl-containing compound comprises a dialkyl butanedicarboxylate.

12. A process in accordance with Claim 7 wherein the carbonyl-containing compound comprises a glycerol ester.

13. A process in accordance with Claim 7 wherein the carbonyl-containing compound comprises a dialkyl glutarate.

14. A process in accordance with Claim 7 wherein the carbonyl-containing compound is selected from the group consisting of dialkyl fumarates, succinates, maleates, and mixtures of any two or more thereof.

15. A process in accordance with Claim 7 wherein the carbonyl-containing compound comprises an alkyl decanoate.

16. A process in accordance with Claim 7 wherein the carbonyl-containing compound comprises an alkyl dodecanoate.

17. A process in accordance with Claim 7 wherein the carbonyl-containing compound is selected from the group consisting of alkyl acetates, propionates, butyrates, valerates, caproates, and mixtures of any two or more thereof.

18. A process in accordance with any of Claims 8-17 said alkyl radical has in the range of 1 up to 6 carbon atoms.

## Patentansprüche

1. Verfahren zur Niedrigdruckhydrierung von Carbonylgruppen enthaltenden Verbindungen zur Herstellung der entsprechenden Alkohole, wobei die Carbonylgruppen enthaltenden Verbindungen die folgende Struktur haben: 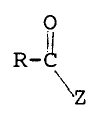 wobei R ein C₁-C₂₀ Alkyl- oder substituierter Alkylrest ist oder ein C₂-C₂₀ Alkenyl- oder Alkynylrest oder ein substituiertes Derivat davon ist, wobei die substituierten Gruppen Ether, Amine, zusätzliche Carbonylgruppen, Arylgruppen, Hydroxylgruppen und Alkoxygruppen einschließen, und Z = OR', wobei R' genauso definiert ist wie R und unabhängig von R ausgewählt ist, mit der Maßgabe, daß R und R' verschieden sind; dieses Verfahren umfaßt das Inkontaktbringen der Carbonylgruppen enthaltenden Verbindungen oder Mischungen von beliebigen zwei oder mehreren davon mit einem Palladium enthaltendem Katalysator auf einem Zinkoxidträger umfassend 0,01 bis zu 20 Gew.-% Palladium, berechnet als das Metall und bezogen auf das Gesamtgewicht des Katalysatoren; wobei das Inkontaktbringen durchgeführt wird in Anwesenheit von Wasserstoff unter Hydrierbedingungen und in der weiteren Anwesenheit von im Bereich von 0,01 bis zu 2 Gew.-% Wasser, bezogen auf das Gesamtgewicht der Reaktanten und Lösungsmittel mit denen der Reaktor befüllt ist.

2. Verfahren nach Anspruch 1, wobei die Hydrierbedingungen eine Temperatur im Bereich von 25 bis zu 400°C und einen Druck im Bereich von 100 bis 10.000 psig (700 - 70.000 kPa) umfassen.

3. Verfahren nach Anspruch 1, wobei der Wasserstoffpartialdruck in den Bereich von 100 bis 10.000 psig (700 - 70.000 kpa) fällt.

4. Verfahren nach Anspruch 1, wobei die Hydrierbedingungen eine Temperatur im Bereich von 100 bis zu 290°C und einen Druck von im Bereich von 100 bis zu 2.500 psig (700 bis 17500 kpa) umfassen.

5. Verfahren nach Anspruch 1, wobei die Stromraumgeschwindigkeit (liquid hourly space velocity) in den Bereich von etwa 0.01 bis zu 100 h⁻¹ fällt.

6. Verfahren nach Anspruch 1, wobei das Gewichtsverhältnis Carbonyl enthaltende Verbindung zu Katalysator in den Bereich von 1:1 bis zu 10.000:1 fällt.

7. Verfahren nach Anspruch 1, wobei die Carbonylgruppen enthaltende Verbindung ausgewählt ist aus der Gruppe bestehend aus:
YO₂C―A―CO₂, (a)
wobei A ein Alkylenrest, ein Alkenylenrest oder ein Alkynylenrest mit 1 bis 20 Kohlenstoffatomen oder ein substituiertes Derivat davon oder ein Cykloalkyl- oder Cykloalkenylrest mit 4-12 Kohlenstoffatomen oder ein substituiertes Derivat davon ist und wobei Y jeweils unabhängig voneinander ein C₁- bis zu C₁₂-Alkyl-, -Alkenyl- oder -Alkynyl-Rest oder substituiertes Derivat davon ist;
B-CO₂Y (b)
wobei B ein Alkyl-, Alkenyl- oder Alkynyl-Rest oder ein substituiertes Derivat von mit 1 bis 20 Kohlenstoffatomen ist und wobei Y wie zuvor definiert ist; 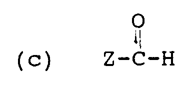 wobei Z ein Alkyl-, Alkenyl- oder Alkynyl-Rest mit 1 bis 20 Kohlenstoffatomen oder ein substituiertes Derivat davon ist, und Mischungen aus beliebigen zwei oder mehreren davon.

8. Verfahren nach Anspruch 7, wobei die Carbonylgruppen enthaltende Verbindung ein Dialkyladipat umfaßt.

9. Verfahren nach Anspruch 7, wobei die Carbonyl enthaltende Verbindung ein Dialkylcyklohexandicarboxylat umfaßt.

10. Verfahren nach Anspruch 7, wobei die Carbonyl enthaltende Verbindung ausgewählt ist aus der Gruppe bestehend aus einem Alkyloleat, einem Alkylstearat, einem Alkyllinoleat, einem Alkyl-α-Eleostearat, einem Alkyl-β-Eleostearat und Mischungen aus beliebigen zwei oder mehreren davon.

11. Verfahren nach Anspruch 7, wobei die Carbonyl enthaltende Verbindung ein Dialkylbutandicarboxylat umfaßt.

12. Verfahren nach Anspruch 7, wobei die Carbonyl enthaltende Verbindung ein Glycerolester umfaßt.

13. Verfahren nach Anspruch 7, wobei die Carbonyl enthaltende Verbindung ein Dialkylglutarat umfaßt.

14. Verfahren nach Anspruch 7, wobei die Carbonyl enthaltende Verbindung ausgewählt ist aus der Gruppe bestehend aus Dialkylfumaraten, Succinaten, Maleaten und aus beliebigen zwei oder mehreren davon.

15. Verfahren nach Anspruch 7, wobei die Carbonyl enthaltende Verbindung ein Alkyldecanoat umfaßt.

16. Verfahren nach Anspruch 7, wobei die Carbonyl enthaltende Verbindung ein Alkyldodecanoat umfaßt.

17. Verfahren nach Anspruch 7, wobei die Carbonyl enthaltende Verbindung ausgewählt ist aus der Gruppe bestehend aus Alkylacetaten, Propionaten, Butyraten, Valeraten, Caproaten und Mischungen aus beliebigen zwei oder mehreren davon.

18. Verfahren nach einem der Ansprüche 8 bis 17, wobei der Akylrest im Bereich von 1 bis 6 Kohlenstoffatome hat.

## Revendications

1. Procédé pour l'hydrogénation basse pression de composés carbonylés pour produire l'alcool correspondant, dans lequel lesdits composés carbonylés ont la structure : 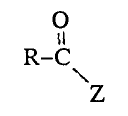 dans laquelle R est un radical alkyle ou alkyle substitué en C₁-C₂₀ ou
un radical alcényle ou alcynyle en C₂-C₂₀ ou un dérivé substitué de celui-ci,
lesdits groupes substitués comprenant des éthers, des amines, des groupes carbonyles supplémentaires, des groupes aryles, des groupes hydroxyles et des groupes alcoxy, et
Z = OR' où R' est défini comme R et est choisi indépendamment de R, à condition que R et R' soient différents,
ledit procédé comprenant la mise en contact desdits composés carbonylés ou de mélanges de deux quelconques ou plus de ceux-ci avec un catalyseur contenant du palladium supporté par de l'oxyde de zinc comprenant 0,01 à 20 % en masse de palladium calculé en métal et sur la base de la masse totale du catalyseur, dans lequel ladite mise en contact est réalisée en présence d'hydrogène dans des conditions d'hydrogénation et en présence également d'eau dans le domaine de 0,01 à 2,0 % en masse sur la base de la masse totale des corps réagissants et du solvant introduits dans le réacteur.

2. Procédé selon la revendication 1, dans lequel lesdites conditions d'hydrogénation comprennent une température située dans le domaine de 25 à 400°C et une pression située dans le domaine de 100 à 10000 psig (700 à 70 000 kPa).

3. Procédé selon la revendication 1, dans lequel la pression partielle de l'hydrogène est située dans le domaine de 100 à 10 000 psig (700 à 70 000 kPa).

4. Procédé selon la revendication 1, dans lequel lesdites conditions d'hydrogénation comprennent une température située dans le domaine de 100 à 290°C et une pression située dans le domaine de 100 à 2 500 psig (700 à 17 500 kPa).

5. Procédé selon la revendication 1, dans lequel la vitesse spatiale horaire liquide est située dans le domaine d'environ 0,01 à 100 h⁻¹.

6. Procédé selon la revendication 1, dans lequel le rapport massique du composé carbonylé au catalyseur est situé dans le domaine de 1:1 à 10 000:1.

7. Procédé selon la revendication 1, dans lequel le composé carbonylé est choisi dans le groupe consistant en :
YO₂C―A―CO₂Y (a)
où A est une portion alkylène, une portion alcénylène ou une portion alcynylène ayant 1 à 20 atomes de carbone, ou un dérivé substitué de celle-ci, ou une portion cycloalkyle ou cycloalcényle ayant 4 à 12 atomes de carbone ou un dérivé substitué de celle-ci, et où chaque Y est indépendamment un radical alkyle, alcényle ou alcynyle en C₁-C₁₂ ou un dérivé substitué de celui-ci,
B―CO₂Y (b)
où B est un radical alkyle, alcényle ou alcynyle, ou un dérivé substitué de celui-ci, ayant 1 à 20 atomes de carbone et
où Y est défini comme ci-dessus, 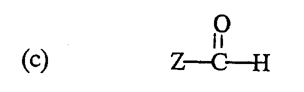 où Z est un radical alkyle, alcényle ou alcynyle ayant 1 à 20 atomes de carbone ou les dérivés substitués de celui-ci, et
des mélanges de deux quelconques ou plus de ceux-ci.

8. Procédé selon la revendication 7, dans lequel le composé carbonylé comprend un adipate de dialkyle.

9. Procédé selon la revendication 7, dans lequel le composé carbonylé comprend un cyclohexanedicarboxylate de dialkyle.

10. Procédé selon la revendication 7, dans lequel le composé carbonylé est choisi dans le groupe consistant en un oléate d'alkyle, un stéarate d'alkyle, un linoléate d'alkyle, un linolénate d'alkyle, un α-éléostéarate d'alkyle, un β-éléostéarate d'alkyle et les mélanges de deux quelconques ou plus de ceux-ci.

11. Procédé selon la revendication 7, dans lequel le composé carbonylé comprend un butanedicarboxylate de dialkyle.

12. Procédé selon la revendication 7, dans lequel le composé carbonylé comprend un ester du glycérol.

13. Procédé selon la revendication 7, dans lequel le composé carbonylé comprend un glutarate de dialkyle.

14. Procédé selon la revendication 7, dans lequel le composé carbonylé est choisi dans le groupe consistant en les fumarates, succinates, maléates de dialkyle et les mélanges de deux quelconques ou plus de ceux-ci.

15. Procédé selon la revendication 7, dans lequel le composé carbonylé comprend un décanoate d'alkyle.

16. Procédé selon la revendication 7, dans lequel le composé carbonylé comprend un dodécanoate d'alkyle.

17. Procédé selon la revendication 7, dans lequel le composé carbonylé est choisi dans le groupe consistant en les acétates, propionates, butyrates, valérates, caproates d'alkyle et les mélanges de deux quelconques ou plus de ceux-ci.

18. Procédé selon l'une quelconque des revendications 8 à 17, dans lequel ledit radical alkyle a de 1 à 6 atomes de carbone.
